# EUROPEAN PATENT APPLICATION

(11) **EP 3 381 283 A1**
(43) Date of publication of application: **03.10.2018**
(21) Application number: 17164200.2
(22) Date of filing: 31.03.2017
(51) Int. Cl.: A01N 31/08, A01N 35/02, A01N 35/04, A01N 37/10, A01N 37/18, A01N 43/90, C07D 493/04, C02F 1/50

(54) **COMPOUNDS FOR THE CONTROL OF BIOFILMS IN INDUSTRIAL EQUIPMENT**

(71) Applicant: Nitto Denko Corporation, Ibaraki-shi, Osaka 567-8680 (JP)
(72) Inventor: Mesnier, Xavier, 1007 Lausanne (CH); Schüwer, Nicolas, 1007 Lausanne (CH); De Visscher, Geofrey, 1071 Chexbres (CH); De Titta, Alexandre, 1028 Préverenges (CH)
(74) Representative: Hoffmann Eitle

(57) **Abstract**

The present invention relates to a method for inhibiting or reducing the growth of a microbial biofilm on a surface, characterised in that the method comprises a step of contacting the biofilm-forming microorganism with a compound according to Formula (1), or a salt or solvate thereof: 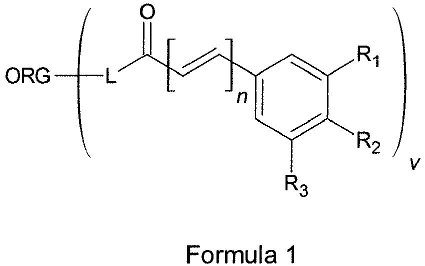 wherein
"ORG" is an organic moiety having from 2 to 20 carbon atoms;
"v" is 1,2, 3, 4, or 5;
"L" provides a covalent link to "ORG" and is selected from O, NH, or is a direct bond from the carbonyl group to "ORG";
"n" is 0 or 1; and
R₁, R₂ and R₃ are independently selected from H, OH and OCH₃ provided that at least one of R₁, R₂ and R₃ is OH.

## Description

### FIELD OF THE INVENTION

This invention provides compounds according to Formula (1) which are useful in treating bacterial biofilms, as well as compositions comprising compounds according to Formula (1) and one or more biocides.

### BACKGROUND OF THE INVENTION

Bacteria communicate with one another using chemical signal molecules. The information supplied by these chemical signal molecules is critical for synchronizing the activities of large groups of bacterial cells. This chemical communication involves producing, releasing, detecting, and responding to small hormone-like molecules, or autoinducers. This process is otherwise known as quorum sensing, and it allows bacteria to monitor the environment for other bacteria and to alter behaviour on a population-wide scale in response to changes in the number and/or species present in a community. Most quorum sensing-controlled processes are beneficial to the population when carried out simultaneously by a large number of cells. Thus, inhibition of quorum sensing can disrupt the health of a bacterial community. If the bacteria are involved in industrial equipment deterioration or leading to malfunctions, inhibition of quorum sensing can have economical value.

Quorum sensing plays a large role in the formation of biofilms. A biofilm is made up of bacteria, often multi-species, embedded in an extracellular matrix of their own making, and further comprises proteins, polysaccharides and DNA. The build-up of the extracellular matrix starts when enough bacteria are present on a surface and quorum sensing mechanisms induces cells to transit from a planktonic to a biofilm or sessile state. When in biofilms, the metabolism and the complexity of the environment change, which oftentimes makes it more difficult to treat the bacteria with biocides.

Biofilm deposition causes a plethora of problems in industrial, biotechnological and medical areas. In the oil industry, for example, the growth of biofilm on equipment can lead to bio-corrosion, biofouling, contamination of oil and gas also called "souring" and degradation of drilling and fracturing fluids for extraction of shale gas. One particular type of bacteria, sulphate reducing bacteria (SRB) can contaminate or "sour" oil reservoir by producing hydrogen sulphide (H₂S) as well as corrode and plug pipes, tanks, filters, valves, pumps, joints and other pieces of equipment used in the oil and gas industries. Additional group of bacteria have been found to cause bio-corrosion and biofouling, such as Iron Reducing Bacteria (IRB), Nitrate Reducing Bacteria (NRB), Acid Producing Bacteria (APB) as well as Archaea, the other domain of prokaryote single-celled microorganisms. These organisms are routinely found in oil production systems and can grow in difficult environments such as high temperature, pressure, salinity. MIC problem is also common in water utilities system and power plants. Additionally, biofilms are a major issue for membranes used in water purification units. Cooling towers and air conditioning systems, air conditioning and humidifying systems are also concerned with microbial biofilms.

Microbial biofilm are becoming increasingly problematic due to widespread practice of enhanced oil recovery based on sea water injection. Sea water contains various types of microorganisms and for this reason, biocides are commonly used. However, it is well known that it is more difficult to kill sessile cells than planktonic cells. Dense biofilms slows or prevent biocide penetration. It is said that a concentration 10 times higher is required to deal with biofilms compared to planktonic cells. Glutaraldehyde and tetrakis(hydroxymethyl)phosphonium sulphate (THPS) are commonly used as biocides. Furthermore, biocide residence time is usually too short to obtain complete eradication. In addition, the prolonged use of the same type of biocide has promoted the surge of biocide resistance which in turn promotes an escalation in biocide concentration used. This is not only more expensive for the industry but there are also great environmental concerns.

Microorganisms have a strong impact in the oil, gas, and water treatment industries and a new strategy is required to solve the pressing issue of biocide resistance and dosage escalation in the fight against biofilms. There is a demand for compounds that can inhibit biofilm formation, or when used together with biocides, potentiate the effect of those biocides. The present invention provides such compounds.

US 8063108 B2 describes a method for modulating microbial quorum sensing by modulating antagonist and agonist compounds, and methods for treating or preventing microbial damages and diseases. Such compounds can be used to prevent or treat biofilms in industrial settings such as oil recovery.

WO 2014/165813 describes systems and methods for controlling microbial growth and activity in gas field or oil field fluid. The method includes the use of two different biocides but does not involve modifying quorum sensing.

US 8846732 describes a composition for effectively inhibiting sulphide production by SRB, which includes a non-oxidizing biocide and a metabolic inhibitor.

US 2011/0152176 introduces activators of bacterial gene regulatory system to block, inhibit or reverse biofilm formation which may be located on industrial surfaces.

### SUMMARY OF THE INVENTION

The inventors have discovered that compounds according to Formula 1 can be used to inhibit or reduce the growth of a microbial biofilm on a surface. Thus, in one aspect the present invention relates to a method for inhibiting or reducing the growth of a microbial biofilm on a surface, characterised in that the method comprises a step of contacting the biofilm-forming microorganism with a compound according to Formula (1), or a salt or solvate thereof: 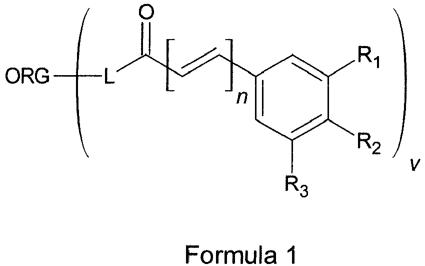 wherein
"ORG" is an organic moiety having from 2 to 20 carbon atoms;
"v" is 1,2, 3, 4, or 5;
"L" provides a covalent link to "ORG" and is selected from O, NH, or is a direct bond from the carbonyl group to "ORG";
"n" is 0 or 1; and
R₁, R₂ and R₃ are independently selected from H, OH and OCH₃ provided that at least one of R₁, R₂ and R₃ is OH.

The inventors have also discovered that compounds according to Formula (1) can be used to potentiate the effect of a biocide. Thus, in one aspect the invention relates to a method for inhibiting or reducing the growth of a microbial biofilm on a surface, characterised in that the method comprises a step of concomitantly contacting the biofilm-forming microorganism with a biocide and a compound according to Formula (1), or a salt or solvate thereof

The present invention also relates to compounds according to Formula (1), as well as compositions comprising compounds of Formula (1). In one aspect the present invention relates to compositions comprising one or more compounds according to Formula (1) and one or more biocides.

### BRIEF DESCRIPTION OF THE DRAWINGS

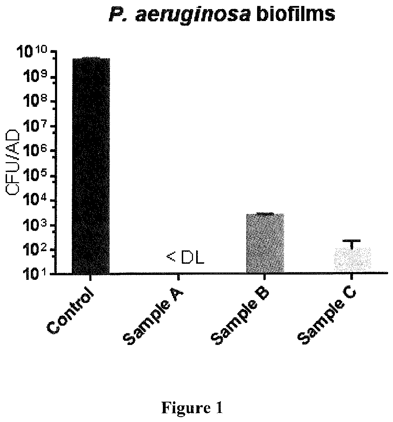

Figure 1 shows that a composition comprising a combination of isosorbide digallate ester and tetracycline (Sample A) has a better biofilm eradicating effect than a composition comprising an equivalent amount of tetracycline alone (Sample B) or a composition comprising equivalent amounts of the individual components tetracycline, isosorbide and gallic acid (Sample C).

### DETAILED DESCRIPTION OF THE INVENTION

### Compounds according to Formula (1)

The inventors have discovered that compounds according to Formula 1 can be used to inhibiting or reducing the growth of a microbial biofilm on a surface: 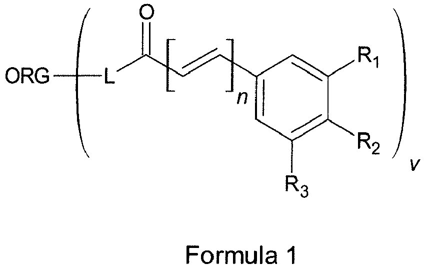 wherein
"ORG" is an organic moiety having from 2 to 20 carbon atoms;
"v" is 1, 2, 3, 4, or 5;
"L" provides a covalent link to "ORG" and is selected from O, NH, or is a direct bond from the carbonyl group to "ORG";
"n" is 0 or 1; and
R₁, R₂ and R₃ are independently selected from H, OH and OCH₃ provided that at least one of R₁, R₂ and R₃ is OH.

In one aspect of the invention, in Formula (1) "v" is 2, 3, or 4, and "L" is O. In another aspect of the invention, in Formula (1) "v" is 2, 3, or 4, and "L" is NH. In another aspect of the invention, in Formula (1) "v" is 2, 3, or 4, and "L" is a direct bond from the carbonyl group to "ORG".

In one aspect of the invention, in Formula (1) "v" is 2, 3, or 4, and "n" is 0. In another aspect of the invention, in Formula (1) "v" is 2, 3, or 4, and "n" is 1.

In one aspect of the invention, "L" is O and "n" is 1. In one aspect of the invention, "L" is O and "n" is 0. In one aspect of the invention, "L" is NH and "n" is 1. In one aspect of the invention, "L" is NH and "n" is 0. In one aspect of the invention, "L" is a direct bond from the carbonyl group to "ORG" and "n" is 1. In one aspect of the invention, "L" is a direct bond from the carbonyl group to "ORG" and "n" is 0.

In one aspect of the invention, "v" is 1, 2, 3, 4, or 5; "L" is O; and "n" is 1. In one aspect of the invention, "v" is 1, 2, 3, 4, or 5; "L" is O; and "n" is 0. In one aspect of the invention, "v" is 1, 2, 3, 4, or 5; "L" is NH; and "n" is 1. In one aspect of the invention, "v" is 1, 2, 3, 4, or 5; "L" is NH; and "n" is 0. In one aspect of the invention, "v" is 1, 2, 3, 4, or 5; "L" is a direct bond from the carbonyl group to "ORG"; and "n" is 1. In one aspect of the invention, "v" is 1, 2, 3, 4, or 5; "L" is a direct bond from the carbonyl group to "ORG"; and "n" is 0.

In a preferred aspect of the invention, when L is O then n, R₁, R₂, and R₃ are as shown in Table 1 (i.e. any one of Embodiments 1-1 to 1-24):

**Table 1**

| **Embodiment** | **L** | **n** | **R₁** | **R₂** | **R₃** |
|---|---|---|---|---|---|
| 1-1 | O | 0 | OH | H | H |
| 1-2 | O | 0 | OH | H | OH |
| 1-3 | O | 0 | OH | H | OCH₃ |
| 1-4 | O | 0 | OH | OH | H |
| 1-5 | O | 0 | OH | OH | OH |
| 1-6 | O | 0 | OH | OH | OCH₃ |
| 1-7 | O | 0 | OH | OCH₃ | H |
| 1-8 | O | 0 | OH | OCH₃ | OH |
| 1-9 | O | 0 | OH | OCH₃ | OCH₃ |
| 1-10 | O | 0 | H | OH | H |
| 1-11 | O | 0 | OCH₃ | OH | H |
| 1-12 | O | 0 | OCH₃ | OH | OCH₃ |
| 1-13 | O | 1 | OH | H | H |
| 1-14 | O | 1 | OH | H | OH |
| 1-15 | O | 1 | OH | H | OCH₃ |
| 1-16 | O | 1 | OH | OH | H |
| 1-17 | O | 1 | OH | OH | OH |
| 1-18 | O | 1 | OH | OH | OCH₃ |
| 1-19 | O | 1 | OH | OCH₃ | H |
| 1-20 | O | 1 | OH | OCH₃ | OH |
| 1-21 | O | 1 | OH | OCH₃ | OCH₃ |
| 1-22 | O | 1 | H | OH | H |
| 1-23 | O | 1 | OCH₃ | OH | H |
| 1-24 | O | 1 | OCH₃ | OH | OCH₃ |

In a further preferred embodiment of the invention, when L is NH then n, R₁, R₂, and R₃ are as shown in Table 2 (i.e. any one of Embodiments 2-1 to 2-24):

**Table 2**

| **Embodiment** | **L** | **n** | **R₁** | **R₂** | **R₃** |
|---|---|---|---|---|---|
| 2-1 | NH | 0 | OH | H | H |
| 2-2 | NH | 0 | OH | H | OH |
| 2-3 | NH | 0 | OH | H | OCH₃ |
| 2-4 | NH | 0 | OH | OH | H |
| 2-5 | NH | 0 | OH | OH | OH |
| 2-6 | NH | 0 | OH | OH | OCH₃ |
| 2-7 | NH | 0 | OH | OCH₃ | H |
| 2-8 | NH | 0 | OH | OCH₃ | OH |
| 2-9 | NH | 0 | OH | OCH₃ | OCH₃ |
| 2-10 | NH | 0 | H | OH | H |
| 2-11 | NH | 0 | OCH₃ | OH | H |
| 2-12 | NH | 0 | OCH₃ | OH | OCH₃ |
| 2-13 | NH | 1 | OH | H | H |
| 2-14 | NH | 1 | OH | H | OH |
| 2-15 | NH | 1 | OH | H | OCH₃ |
| 2-16 | NH | 1 | OH | OH | H |
| 2-17 | NH | 1 | OH | OH | OH |
| 2-18 | NH | 1 | OH | OH | OCH₃ |
| 2-19 | NH | 1 | OH | OCH₃ | H |
| 2-20 | NH | 1 | OH | OCH₃ | OH |
| 2-21 | NH | 1 | OH | OCH₃ | OCH₃ |
| 2-22 | NH | 1 | H | OH | H |
| 2-23 | NH | 1 | OCH₃ | OH | H |
| 2-24 | NH | 1 | OCH₃ | OH | OCH₃ |

In still a further preferred embodiment of the invention, when L is a direct bond from the carbonyl group to "ORG" then n, R₁, R₂, and R₃ are as shown in Table 3 (i.e. any one of Embodiments 3-1 to 3-24):

**Table 3**

| **Embodiment** | **L** | **n** | **R₁** | **R₂** | **R₃** |
|---|---|---|---|---|---|
| 3-1 | direct bond | 0 | OH | H | H |
| 3-2 | direct bond | 0 | OH | H | OH |
| 3-3 | direct bond | 0 | OH | H | OCH₃ |
| 3-4 | direct bond | 0 | OH | OH | H |
| 3-5 | direct bond | 0 | OH | OH | OH |
| 3-6 | direct bond | 0 | OH | OH | OCH₃ |
| 3-7 | direct bond | 0 | OH | OCH₃ | H |
| 3-8 | direct bond | 0 | OH | OCH₃ | OH |
| 3-9 | direct bond | 0 | OH | OCH₃ | OCH₃ |
| 3-10 | direct bond | 0 | H | OH | H |
| 3-11 | direct bond | 0 | OCH₃ | OH | H |
| 3-12 | direct bond | 0 | OCH₃ | OH | OCH₃ |
| 3-13 | direct bond | 1 | OH | H | H |
| 3-14 | direct bond | 1 | OH | H | OH |
| 3-15 | direct bond | 1 | OH | H | OCH₃ |
| 3-16 | direct bond | 1 | OH | OH | H |
| 3-17 | direct bond | 1 | OH | OH | OH |
| 3-18 | direct bond | 1 | OH | OH | OCH₃ |
| 3-19 | direct bond | 1 | OH | OCH₃ | H |
| 3-20 | direct bond | 1 | OH | OCH₃ | OH |
| 3-21 | direct bond | 1 | OH | OCH₃ | OCH₃ |
| 3-22 | direct bond | 1 | H | OH | H |
| 3-23 | direct bond | 1 | OCH₃ | OH | H |
| 3-24 | direct bond | 1 | OCH₃ | OH | OCH₃ |

In one aspect of the invention, "v" is 1 and L, n, R₁, R₂, and R₃ are any one of the Embodiments 1-1 to 1-24, 2-1 to 2-24, or 3-1 to 3-24 as shown in Table 1, Table 2 or Table 3, respectively (collectively: "v=1 embodiment"). In this embodiment of the invention the compound of Formula 1 has only one moiety satisfying the structure within the round brackets of Formula 1.

In another aspect of the invention, "v" is 2 and L, n, R₁, R₂, and R₃ are any one of the Embodiments 1-1 to 1-24, 2-1 to 2-24, or 3-1 to 3-24 as shown in Table 1, Table 2 or Table 3, respectively (collectively: "v=2 embodiment"). In this embodiment of the invention the compound of Formula 1 has two moieties that independently satisfy the structure within the round brackets of Formula 1, and each of these structures may be the same or different.

In still another aspect of the invention, "v" is 3 and L, n, R₁, R₂, and R₃ are any one of the Embodiments 1-1 to 1-24, 2-1 to 2-24, or 3-1 to 3-24 as shown in Table 1, Table 2 or Table 3, respectively (collectively: "v=3 embodiment"). In this embodiment of the invention the compound of Formula 1 has three moieties that independently satisfy the structure within the round brackets of Formula 1. Thus, each of the three structures may be the same of different from one another.

In a further aspect of the invention, "v" is 4 and L, n, R₁, R₂, and R₃ are any one of the Embodiments 1-1 to 1-24, 2-1 to 2-24, or 3-1 to 3-24 as shown in Table 1, Table 2 or Table 3, respectively (collectively: "v=4 embodiment"). In this embodiment of the invention the compound of Formula 1 has four moieties that independently satisfy the structure within the round brackets of Formula 1. Thus, each of the four structures may be the same of different from one another.

In yet a further aspect of the invention, "v" is 5 and L, n, R₁, R₂, and R₃ are any one of the Embodiments 1-1 to 1-24, 2-1 to 2-24, or 3-1 to 3-24 as shown in Table 1, Table 2 or Table 3, respectively (collectively: "v=5 embodiment"). In this embodiment of the invention the compound of Formula 1 has five moieties that independently satisfy the structure within the round brackets of Formula 1. Thus, each of the five structures may be the same of different from one another.

Without being bound by theory, it is believed that the compounds according to Formula (1) derive their biofilm-disrupting property from the moiety defined within the round brackets. The purpose of "ORG" in Formula (1) is therefore to facilitate delivery of the moiety defined within the round brackets. For the purpose of the present invention, "ORG" is not particularly limited other than it must be an organic moiety having from 2 to 20 carbon atoms. "ORG" can be modified as required to provide 1, 2, 3, 4, or 5 of the moieties defined within the round brackets of Formula (1) (i.e. v=1, 2, 3, 4, or 5). "ORG" can also be modified to tailor the solubility properties of the compound according to Formula (1) depending on the environmental conditions where it is intended to be used to inhibit or reduce the growth of a microbial biofilm (e.g. pH, temperature, solvent system, etc.).

In one aspect of the invention "ORG" is a linear or branched C2-C5 alkane, linear or branched C2-C5 alkene, or linear or branched C2-C5 alkyne that, in addition to the moiety satisfying the structure within the round brackets of Formula 1, may optionally be substituted with one or more substituents selected from -OH; -NH₂; -NH₃⁺X where X is F, Cl, Br or I; a halogen selected from F, Cl, Br or I; -NO₂; a C6-C10 aryl which is linked to the alkane, alkene or alkyne via a linking group selected from direct bond, -O-, -CO-, -COO-, -OCOO-, -COOCH₂-, -COOC₂H₄-, -COOCH=CH-, -NH-, -CONH-, -COONH-, -CSO-, or -CSS-; a 5 to 10 membered saturated, unsaturated or aromatic heterocycle containing 1 to 4 heteroatoms selected from O, N and S which is linked to the alkane, alkene or alkyne via a linking group selected from direct bond, -O-, -CO-, -COO-, -OCOO-, -COOCH₂-, -COOC₂H₄-,-COOCH=CH-, -NH-, -CONH-, -COONH-, -CSO-, or -CSS-; and wherein the aforementioned aryl and heterocycle groups may optionally carry one, two or three substituents independently selected from -OH, F, Cl, Br, I, -NO₂, -NH₂, -NH₃⁺X where X is F, Cl, Br or I. In a preferred embodiment, the optionally substituted C6-10 aryl is benzoic acid or o-, m-, or p-chlorobenzoic acid linked to the alkane, alkene or alkyne via a linking group selected from direct bond, -O-, -CO-, -COO-, -OCOO-, -COOCH₂-, -COOC₂H₄-, -COOCH=CH-, -NH-, - CONH-, -COONH-, -CSO-, or -CSS-.

In a preferred embodiment, "ORG" is a linear or branched C2-C5 alkane which may optionally be substituted as described above. In a further preferred embodiment, "ORG" is a linear or branched C2-C5 alkane which may optionally be substituted with a C6-C10 aryl as described above, preferably a C6 aryl, and if present is linked to the alkane via a linking group selected from direct bond, -O-, -CO-, -COO-, -OCOO-, -COOCH₂-, -COOC₂H₄-,-COOCH=CH-, -NH-, -CONH-, -COONH-, -CSO-, or -CSS-.

In a preferred embodiment of the invention, "ORG" is a linear or branched C2-C5 alkane which may optionally be substituted as described above and is covalently linked to one, two, three, four or five moieties selected from the Embodiments 1-1 to 1-24, 2-1 to 2-24, and 3-1 to 3-24 as shown in Table 1, Table 2 and Table 3, respectively. In a preferred embodiment, "ORG" is a linear or branched C2, C3, C4 or C5 alkane which may optionally be substituted as described above and which is covalently linked to two such moieties (i.e. the v=2 embodiment). In a further preferred embodiment, "ORG" is a linear or branched C3, C4 or C5 alkane which may optionally be substituted as described above and is covalently linked to three such moieties (i.e. the v=3 embodiment). In still a further preferred embodiment, "ORG" is a linear or branched C4 or C5 alkane which may optionally be substituted as described above and is covalently linked to four such moieties (i.e. the v=4 embodiment).

In another aspect of the invention "ORG" is a saturated C5-C6 monocyclic or bicyclic heterocycle having one or two oxygen heteroatoms and that, in addition to the moiety satisfying the structure within the round brackets of Formula 1, may optionally be substituted with one or more substituents selected from -OH; -NH₂; -NH₃⁺X where X is F, Cl, Br or I; a halogen selected from F, Cl, Br or I; -NO₂; a C6-C10 aryl which is linked to the alkane, alkene or alkyne via a linking group selected from direct bond, -O-, -CO-, -COO-, -OCOO-,-COOCH₂-, -COOC₂H₄-, -COOCH=CH-, -NH-, -CONH-, -COONH-, -CSO-, or -CSS-; a 5 to 10 membered saturated, unsaturated or aromatic heterocycle containing 1 to 4 heteroatoms selected from O, N and S which is linked to the alkane, alkene or alkyne via a linking group selected from direct bond, -O-, -CO-, -COO-, -OCOO-, -COOCH₂-, -COOC₂H₄-,-COOCH=CH-, -NH-, -CONH-, -COONH-, -CSO-, or -CSS-; and wherein the aforementioned aryl and heterocycle groups may optionally carry one, two or three substituents independently selected from -OH, F, Cl, Br, I, -NO₂, -NH₂, -NH₃⁺X where X is F, Cl, Br or I. In a preferred embodiment, the optionally substituted C6-10 aryl is benzoic acid or o-, m-, or p-chlorobenzoic acid linked to the C5-C6 monocyclic or bicyclic heterocycle via a linking group selected from direct bond, -O-, -CO-, -COO-, -OCOO-, -COOCH₂-, -COOC₂H₄-,-COOCH=CH-, -NH-, -CONH-, -COONH-, -CSO-, or -CSS-.

In a preferred embodiment, "ORG" is a saturated C5 monocyclic heterocycle having one oxygen heteroatom. (i.e. six-membered ring) and which may optionally be substituted as described above. In a further preferred embodiment, "ORG" is a C6 bicyclic heterocycle having two oxygen heteroatoms (e.g. a fused bicycle with each ring having one oxygen heteroatom) and which may optionally be substituted as described above. In either case it is preferred that the substituent, if present, is selected from -OH; -NH₂; -NH₃⁺X where X is F, Cl, Br or I; C6-C10 aryl as described above, preferably a C6 aryl, and if present is linked to the alkane via a linking group selected from direct bond, -O-, -CO-, -COO-, -OCOO-,-COOCH₂-, -COOC₂H₄-, -COOCH=CH-, -NH-, -CONH-, -COONH-, -CSO-, or -CSS-.

In a preferred embodiment of the invention, "ORG" is a saturated C5-C6 monocyclic or bicyclic heterocycle having one or two oxygen heteroatoms which may optionally be substituted as described above and which is covalently linked to one, two, or three moieties selected from the Embodiments 1-1 to 1-24, 2-1 to 2-24, and 3-1 to 3-24 as shown in Table 1, Table 2 and Table 3, respectively. In a preferred embodiment, "ORG" is a saturated C5 monocyclic heterocycle having one oxygen heteroatom. (i.e. six-membered ring) and which may optionally be substituted as described above and which is covalently linked to one such moiety (i.e. the v=1 embodiment). In a further preferred embodiment, "ORG" is a saturated C5 monocyclic heterocycle having one oxygen heteroatom. (i.e. six-membered ring) and which may optionally be substituted as described above and which is covalently linked to two such moieties (i.e. the v=2 embodiment). In a further preferred embodiment "ORG" is a C6 bicyclic heterocycle having two oxygen heteroatoms (e.g. a fused bicycle with each ring having one oxygen heteroatom) which may optionally be substituted as described above and which is covalently linked to one such moiety (i.e. the v=1 embodiment). In still a further preferred embodiment, "ORG" is a C6 bicyclic heterocycle having two oxygen heteroatoms (e.g. a fused bicycle with each ring having one oxygen heteroatom) which may optionally be substituted as described above and which is covalently linked to two such moieties (i.e. the v=2 embodiment).

The following structures are preferred examples of "ORG" for the purpose of the present invention.

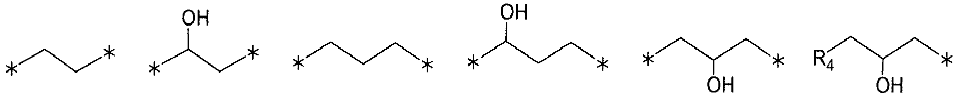

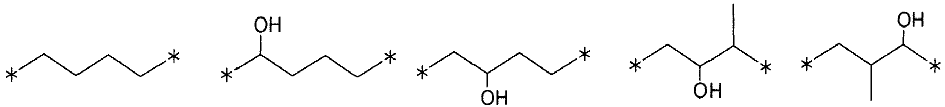

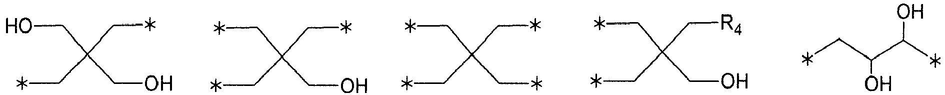

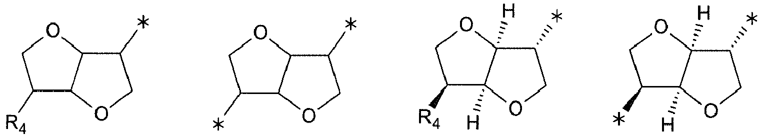

In each case, the asterisk denotes the position of "L" within the round bracket in Formula (1). A structure having two asterisks is one where v=2 and thus will have two moieties independently satisfying the structure within the round brackets of Formula (1). Likewise a structure having three or four asterisks is one where v=3 and v=4, respectively.

In the structures for "ORG" above, R₄ is a substituent selected from -OH; -NH₂; -NH₃⁺X where X is F, Cl, Br or I; a halogen selected from F, Cl, Br or I; -NO₂; a C6-C10 aryl which is linked to the alkane, alkene or alkyne via a linking group selected from direct bond, -O-, -CO-, -COO-, -OCOO-, -COOCH₂-, -COOC₂H₄-, -COOCH=CH-, -NH-, -CONH-, -COONH-,-CSO-, or -CSS-; a 5 to 10 membered saturated, unsaturated or aromatic heterocycle containing 1 to 4 heteroatoms selected from O, N and S which is linked to the alkane, alkene or alkyne via a linking group selected from direct bond, -O-, -CO-, -COO-, -OCOO-,-COOCH₂-, -COOC₂H₄-, -COOCH=CH-, -NH-, -CONH-, -COONH-, -CSO-, or -CSS-; and wherein the aforementioned aryl and heterocycle groups may optionally carry one, two or three substituents independently selected from -OH, F, Cl, Br, I, -NO₂, -NH₂, -NH₃⁺X where X is F, Cl, Br or I.

Preferred compounds according to Formula (1) for the purpose of the present invention include the following:

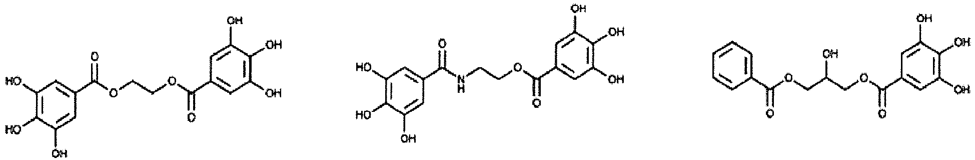

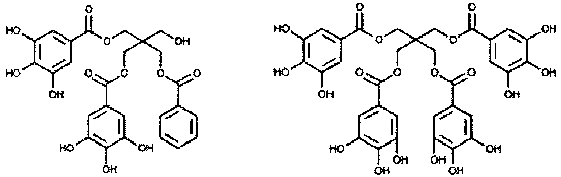

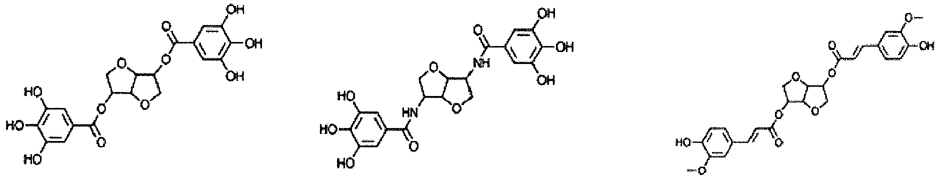

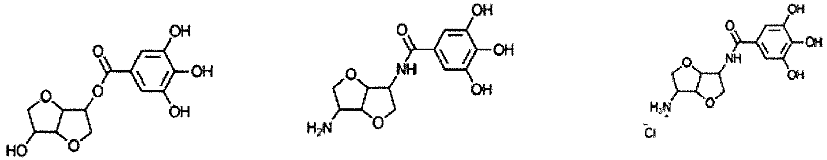

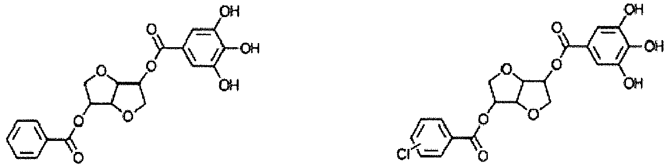

Of the dianhydrohexitol-based structures shown above, the isosorbide-, isoidide- or isomannide-stereo configuration can be used for the invention. Of these, the isosorbide-configuration is preferable.

It is to be understood that any disclosure herein with respect to the compounds of Formula (1) also extends to tautomers, salts, polymorphs, and solvates thereof. "Salts", as used herein, are salts that retain the desired biological activity of the parent compound and do not impart undesired toxicological effects. Salt forms include various crystalline polymorphs as well as the amorphous form of the different salts. The salts can be formed with metal or organic counterions and include, but are not limited to, alkali metal salts such as sodium or potassium; alkaline earth metal salts such as magnesium or calcium; and ammonium or tetraalkyl ammonium salts. The salts can be organic or inorganic in nature. Representative salts include acetate, benzenesulfonate, benzoate, bicarbonate, bisulfate, bitartrate, borate, bromide, calcium edetate, camsylate, carbonate, chloride, clavulanate, citrate, dihydrochloride, edetate, edisylate, estolate, esylate, fumarate, gluceptate, gluconate, glutamate, glycollylarsanilate, hexylresorcinate, hydrabamine, hydrobromide, hydrochloride, hydroxynaphthoate, iodide, isethionate, lactate, lactobionate, laurate, malate, maleate, mandelate, mesylate, methylbromide, methylnitrate, methylsulfate, monopotassium maleate, mucate, napsylate, nitrate, N-methylglucamine, oxalate, pamoate (embonate), palmitate, pantothenate, phosphate/diphosphate, polygalacturonate, potassium, salicylate, sodium, stearate, subacetate, succinate, tannate, tartrate, teoclate, tosylate, triethiodide, trimethylammonium and valerate. "Solvates", as used herein, are addition complexes in which the compound is combined with an acceptable co-solvent in some fixed proportion. Co-solvents include, but are not limited to, ethyl acetate, lauryl lactate, myristyl lactate, cetyl lactate, isopropyl myristate, methanol, ethanol, 1-propanol, isopropanol, 1-butanol, isobutanol, tert-butanol, acetone, methyl ethyl ketone, acetonitrile, benzene, toluene, xylene(s), ethylene glycol, dichloromethane, 1,2-dichloroethane, *N*-methylformamide, *N,N-*dimethylformamide, *N*-methylacetamide, pyridine, dioxane, and diethyl ether. The term 'hydrate' is employed when the co-solvent is water.

### Treatment of bacterial biofilms

The compounds having Formula (1) as described above are suitable for use in the treatment of biofilm and the prevention of its formation. In one embodiment of the invention, the compounds having Formula (1) are used to reduce the effective dose of biocide required to eliminate a biofilm. In another embodiment, the compounds having Formula (1) are used to increase efficacy of a biocide without increasing its dosage. In another embodiment, the compounds having Formula (1) are used to prevent a biofilm from forming, with or without a biocide.

The term "*biofilm*" is to be understood as having its ordinary meaning. A biofilm is a sessile community of microorganisms characterized by cells that are attached to a substratum or interface or to each other, that are embedded in a matrix of extracellular polymers (more specifically extracellular polymers that they have produced), and that exhibit an altered phenotype with respect to growth rate and gene transcription (for example as compared to their "non-biofilm" or free-floating or planktonic counterparts).

The biofilms that may be treated in accordance with the present invention are not limited in terms of the microorganisms that they contain or in the combination of microorganisms that they contain. The biofilm may comprise any class, genus or species of microorganism, namely any microorganism that may form a biofilm. The microorganisms included in the biofilm may include bacteria and archaea whose quorum sensing mechanisms are discussed below. Microorganism may be aerobic or anaerobic, gram+, gram- or gram test non-responsive.

In one aspect, the invention relates to a method for inhibiting or reducing the growth of a microbial biofilm caused by microorganisms that communicate by quorum sensing. Quorum sensing is the cell to cell communication and signaling system used by some microorganisms, particularly bacteria. Different bacteria use different signal molecules for quorum sensing. In one aspect, the invention relates to a method for inhibiting or reducing the growth of biofilm caused by bacteria and/or archaea that use autoinducer-1 type signaling molecules, otherwise referred to as acyl-homoserine lactone (AHLs) (see Miller et al., Annu. Rev. Microbiol. 55 (2001) 165-99; Lillicrap et al., Chemosphere. 164 (2016) 52-58). In another aspect, the invention relates to a method for inhibiting or reducing the growth of a bacterial biofilm caused by bacteria that use peptides as signaling molecules (Waters and Bassler, Annu. Rev. Cell Dev. Biol. 21 (2005) 319-346). In still another aspect, the invention relates to a method for inhibiting or reducing the growth of a bacterial biofilm caused by bacteria that use autoinducer-2 (a furanosyl borate diester) as a signal molecule (see von Bodman et al., J. Bacteriol. 190 (2008) 4377-4391; Miller et al., Annu. Rev. Microbiol. 55 (2001) 165-99). In a further aspect the invention relates to a method for inhibiting or reducing the growth of a bacterial biofilm caused by bacteria that use quinolone compounds such as 4-hydroxy-2-alkylquinolines (HAQs) as signaling molecules (see Sifri et al., Clin. Infect. Dis. 47 (2008) 1070-1076).

In one aspect, the invention relates to a method for inhibiting or reducing the growth of a microbial biofilm caused by microorganisms categorized by their metabolism that leads to microbial-induced corrosion.

For example, in a preferred embodiment the invention is directed to a method for inhibiting or reducing the growth of a microbial biofilm caused by sulphate reducing microorganisms. These are bacteria and archaea that have the ability to use sulphate, sulphite, sulfure, thiosulfate, as a terminal electron acceptor and/or iron (stainless steel, carbon steel, high-alloy steel) and oxidized iron (Fe³⁺, Fe²⁺) as electron donor. Such microorganisms may be aerobic or anaerobic, gram+, gram- or gram test non-responsive. Examples include:
Deltaproteobacteria such as Genus *Desulfobulbaceae,* Genus *Desulfobacteraceae, Desulfobacterium anilini, Desulfarculus baarsii,* Genus *Syntrophobacteraceae, Desulfomonile limimaris, Desulfomonile tiedjei, Desulfomonile* spp., *Desulfovibrio* spp., *Desulfonatronum lacustre, Desulfonatronum thiodismutans, Desulfonatronum cooperativum, Desulfonatronum* spp., *Desulfonatronovibrio hydrogenovorans, Desulfonatronovibrio* spp., *Desulfomicrobium* spp., *Desulfocaldus terraneus, Desulfocaldus* spp., and *Desulfohalobium* spp.;
Nitrospirae such as *Thermodesulfovibrio islandicus, Thermodesulfovibrio yellowstonii,* and *Thermodesulfovibrio* spp.;
Clostridia such as, *Desulfosporomusa polytropa, Desulfosporomusa* spp., *Desulfosporosinus orientis, Desulfosporosinus youngii, Desulfosporosinus meridiei, Desulfosporosinus* spp., and *Desulfotomaculum* spp.;
Thermodesulfobiaceae such as *Thermodesulfobium narugense,* and *Thermodesulfobium* spp.;
Thermodesulfobacteria such as *Thermodesulfobacterium hveragerdense, Thermodesulfobacterium thermophilum, Thermodesulfobacterium commune, Thermodesulfobacterium hydrogeniphilum, Thermodesulfobacterium* spp., *Thermodesulfatator indicus,* and *Thermodesulfatator* spp.;
Euryarchaeota such as *Archaeoglobus profundus, Archaeoglobus veneficus, Archaeoglobus lithotrophicus,* and *Archaeoglobus* spp.; and
Crenarchaeota such as *Thermocladium modestius, Thermocladium* spp., *Caldivirga maquilingensis, Caldivirga* spp., and *Pseudomonas aeroginosa*

Other microorganisms involved in microbial-induced corrosion which can be treated for the purposes of the present invention are methanogenic-capable bacteria/archaea such as *Methanoplanus* spp, *Methanobacterium* spp., *Methanothermobacter* spp., *Methanocalculus* spp., *Methanosarcina* spp. or *Methanohalophiluus* spp., acid-producing bacteria/archaea such as *Thiobacillus* spp., nitrate and nitrite-reducing bacteria such as *Sulfurospirillum* spp. and *Geobaccilus* spp. and manganese reducing-bacteria such as *Deferribacter* spp.

In a preferred embodiment the biofilm to be treated is one containing *Pseudomonas aeruginosa.*

In one aspect, the invention relates to a method for inhibiting or reducing the growth of a microbial biofilm caused by Gram-positive bacteria and/or Gram-negative bacteria.

The term 'Gram-positive bacteria' is known in the art and refers to bacteria characterized by having as part of their cell wall structure peptidoglycan as well as polysaccharides and/or teichoic acids and are characterized by their blue-violet colour reaction in the Gram-staining procedure. Representative Gram-positive bacteria for the purpose of this invention include: *Actinomyces* spp., *Bacillus anthracis, Bifidobacterium* spp., *Clostridium botulinum, Clostridium perfringens, Clostridium* spp., *Clostridium tetani, Corynebacterium diphtheriae, Corynebacterium jeikeium, Enterococcus faecalis, Enterococcus faecium, Erysipelothrix rhusiopathiae, Eubacterium* spp., *Gardnerella vaginalis, Gemella morbillorum, Leuconostoc* spp., *Mycobacterium abcessus, Mycobacterium avium complex, Mycobacterium chelonae, Mycobacterium fortuitum, Mycobacterium haemophilium, Mycobacterium kansasii, Mycobacterium leprae, Mycobacterium marinum, Mycobacterium scrofulaceum, Mycobacterium smegmatis, Mycobacterium terrae, Mycobacterium tuberculosis, Mycobacterium ulcerans, Nocardia* spp., *Peptococcus niger, Peptostreptococcus* spp., *Proprionibacterium* spp., *Staphylococcus aureus, Staphylococcus auricularis, Staphylococcus capitis, Staphylococcus cohnii, Staphylococcus epidermidis, Staphylococcus haemolyticus, Staphylococcus hominis, Staphylococcus lugdanensis, Staphylococcus saccharolyticus, Staphylococcus saprophyticus, Staphylococcus schleiferi, Staphylococcus similans, Staphylococcus warneri, Staphylococcus xylosus, Streptococcus agalactiae* (group B *streptococcus), Streptococcus anginosus, Streptococcus bovis, Streptococcus canis, Streptococcus equi, Streptococcus milleri, Streptococcus mitior, Streptococcus mutans, Streptococcus pneumoniae, Streptococcus pyogenes* (group A *streptococcus*), *Streptococcus salivarius,* and *Streptococcus sanguis.*

The term "Gram-negative bacteria" is likewise known in the art and is a term for bacteria characterized by the presence of a double membrane surrounding each bacterial cell. Representative Gram-negative bacteria for the purpose of the present invention include *Acinetobacter calcoaceticus, Actinobacillus actinomycetemcomitans, Aeromonas hydrophila, Alcaligenes xylosoxidans, Bacteroides, Bacteroides fragilis, Bartonella bacilliformis, Bordetella* spp., *Borrelia burgdorferi, Branhamella catarrhalis, Brucella* spp., *Campylobacter* spp., *Chlamydia pneumoniae, Chlamydia psittaci, Chlamydia trachomatis, Chromobacterium violaceum, Citrobacter* spp., *Eikenella corrodens, Enterobacter aerogenes, Escherichia coli, Flavobacterium meningosepticum, Fusobacterium* spp., *Haemophilus influenzae, Haemophilus* spp., *Helicobacter pylori, Klebsiella* spp., *Legionella* spp., *Leptospira* spp., *Moraxella catarrhalis, Morganella morganii, Mycoplasma pneumoniae, Neisseria gonorrhoeae, Neisseria meningitidis, Pasteurella multocida, Plesiomonas shigelloides, Prevotella* spp., *Proteus* spp., *Providencia rettgeri, Pseudomonas aeruginosa, Pseudomonas* spp., *Rickettsia prowazekii, Rickettsia rickettsii, Rochalimaea* spp., *Salmonella* spp., *Salmonella typhi, Serratia marcescens, Shigella* spp., *Treponema carateum, Treponema pallidum, Treponema pallidum endemicum, Treponema pertenue, Veillonella* spp., *Vibrio cholerae, Vibrio vulnificus, Yersinia enterocolitica, Yersinia pestis., Desulfovibrio spp. Desulfovibrio vulgaris, Desulfovibrio desulphuricans, Caldilineaceae, Incertae Sedis XV, Desulfuromonaceae, Incertae Sedis IV, Flexibacteraceae,* and *Thermodesulfobiaceae, Anaerolineacea*

The bacteria and archaea may be aerobic or anaerobic. The bacteria may be pathogenic or non-pathogenic. It is particularly surprising that the compounds according to Formula (1) are able to disrupt the formation of a biofilm and improve the efficacy of a biocide to kill bacteria and archaea in biofilms. In one embodiment, biofilms comprising or consisting of SRB, are targets for the present invention. In one embodiment, the compound according to Formula (1) is isosorbide digallate ester and the biofilm to be treated or prevented is one comprising *SRB.* Biofilms may also contain fungi, algae, archaea and other organisms such as parasitic protozoa in addition to the bacteria. Such mixed colony biofilms are also treatable according to the methods described herein.

In one embodiment of the invention the compounds according to Formula (1), optionally together with a biocide, are used to inhibit the growth of a biofilm or reduce an existing biofilm.

The term "*inhibit*" and variations thereof as used herein means complete or partial inhibition of biofilm formation and/or development. Inhibition may be effected by treating microorganisms before they have had a chance to form a biofilm or while a biofilm is forming. Further, inhibition may be permanent or temporary. The inhibition may be to an extent (in magnitude and/or spatially), and/or for a time, sufficient to produce the desired effect. Inhibition may be prevention, retardation, or otherwise hindrance of biofilm formation or development. Inhibition of the formation or development of a biofilm by compounds according to Formula (1) can be assessed by measuring biofilm mass or microbial growth in the presence and absence of such compounds. The formation or development of a biofilm can be inhibited by a compound according to Formula (1) by at least about 10%, 15%, 20%, 25%, 30%, 35%, 40%, 45%, 50%, 55%, 60%, 65%, 70%, 75%, 80%, 85%, 90%, 95% or more compared to the formation or development of a biofilm that is not exposed to a compound according to Formula (1).

The term "*reduce*" and variations thereof as used herein means reducing the biomass of an area of a biofilm exposed to an effective amount of a compound according to Formula (1) as compared to the biofilm biomass of the area immediately before exposure to a compound according to Formula (1). In some embodiments the "biomass" is the mass of cells present in the area of biofilm in addition to the extracellular polymeric substance (EPS) of the biofilm matrix. In some embodiments the "biomass" is only the mass of cells present in the area of biofilm (that is, the mass of the EPS is not counted as "biomass"). In some embodiments the biomass of the area of a biofilm exposed to an effective amount of a compound according to Formula (1) is at least 10% less than the biofilm biomass of the area immediately before exposure to the compound, for example, at least 20%, at least 30%, at least 40%, at least 50%, at least 60%, at least 70%, at least 80%, at least 90%, at least 95%, at least 98%, or at least 99% less than the biofilm biomass of the area immediately before exposure to the compound according to Formula (1). While it is to be understood that a biofilm can be treated on a surface of any area, in the present invention assessing the reduction of biofilm is preferably performed on one or more samples of the surface area that is treated. In some embodiments the surface area assessed is 10⁻⁵ m²; in other embodiments the surface area assessed is 10⁻⁴ m², 10⁻³ m², or 10⁻² m². In some embodiments a biofilm whose biomass has been reduced by at least 95% is deemed to have been "eradicated". One way to assess change in biomass is by a method comprising the steps of: i) washing the area of biofilm to remove non-adherent (planktonic) microorganisms, ii) assessing the area of biofilm biomass (i.e. the biomass immediately before exposure to a compound according to Formula (1)), iii) exposing the area of biofilm (or an otherwise identical area) to an effective amount of a compound of formula (1) for a period of time (for example, 24 hours), iv) washing the biofilm to remove non-adherent (planktonic) microorganisms, and v) assessing the area of biofilm biomass to obtain the post-exposure biomass. The size, structure, integrity, and number of microbes in a biofilm can be analysed by any convenient method known in the art such as transmission electronic microscopy.

The biofilm to be treated may be present on a surface. The surface is not limited and includes any surface on which a microorganism may occur. The surface may be biotic or abiotic, and inanimate (or abiotic) surfaces include any such surface which may be exposed to microbial contact or contamination. Thus particularly included are industrial water system surfaces that are exposed to microbial contact or contamination such as a papermaking water system; a cooling water system such as a cooling tower, an open and/or closed loop cooling unit; an industrial raw water system; a drinking water distribution system; a sanitizing drinking water system; an oil production or recovery system such as an oil field water system or a system that comes into contact with drilling fluids; a fuel storage and/or transport system; a metal working system; a heat exchanger; a reactor; equipment used for storing and handling liquids; a boiler; a steam generating unit; a radiator; a flash evaporating unit; a refrigeration unit; reverse osmosis equipment; an ion selective membrane; a gas scrubbing unit; a blast furnace; a sugar evaporating unit; a steam power plant; a geothermal unit; a nuclear cooling unit; a water treatment unit; a pool recirculating unit; a mining circuit; a closed loop heating unit; a system having a surface that comes into contact with machining fluids used in operations such as drilling, boring, milling, reaming, drawing, broaching, turning, cutting, sewing, grinding, thread cutting, shaping, spinning and rolling; a system having a surface that comes into contact with hydraulic fluids; and ; a system having a surface that comes into contact with cooling fluids..

Whether the compounds according to Formula (1) are to be used for eradicating biofilms or preventing its appearance, the compound can be used alone or as a formulation comprising the compound. In one aspect of the invention one or more compounds according to Formula (1) are compounded with other commonly used industrial compounds such as corrosion inhibitors, scale inhibitors, demulsifiers, defoamers, surfactants and the like that are used to treat e.g. water in water injection systems, storage tanks, pipes membranes and other industrial equipment such as that employed in the oil, gas, energy, paper and water treatment industries.

When the compounds according to Formula (1) are used to treat a biofilm on a surface, the compounds can be formulated as a liquid, for ease of handling particularly for use in systems where automatic handling is used. When formulated as a liquid the compounds can be formulated with propylene glycol or other similar ingredients such as glycerol and water. The bacteria-containing medium (e.g. water containing planktonic bacteria) or biofilm to be treated can be contacted in either intermittent (*i.e.,* batch) or continuous fashion. Preferably, in the method of the present invention the compound according to Formula (1) is used at a concentration of 0.1 to 1,000,000 µM, preferably from 1 to 100,000 µM, and even more preferably from 10 to 10,000 µM. When more than one compound according to Formula (1) is used at the same time, then the preferred concentration ranges above refer to each compound according to Formula (1) that is being used. In one embodiment, the bacteria-containing medium (e.g. water containing planktonic bacteria) or biofilm is treated for between 1 minute and 2 days, preferably between 10 minutes and 1 day, preferably between 1 hour and 15 hours, more preferably less than 10 hours. In another embodiment a surface is treated for 2 days or more, 1 week or more, 1 month or more, or continuously to prevent or hinder the formation of a biofilm. In this aspect "continuously" means for as long as there is a threat of a biofilm forming.

The invention also relates to a method for inhibiting or reducing the growth of a microbial biofilm on a surface, characterised in that the method comprises a step of concomitantly contacting the biofilm-forming microorganism with a biocide and a compound according to Formula (1), or a salt or solvate thereof. For this aspect of the present invention, the biocide is understood to be something other than a compound according to Formula (1). The biocide may be any suitable biocide typically used against biofilm-forming microorganisms. In one embodiment the biocide is a mixture of two or more different biocides. The biocide may be soluble to water, or it may have low solubility to water or it may even be non-water-soluble. It is advantageous that the biocide does not react with compounds of Formula (1). Preferably, in the method of the present invention the biocide is used at a concentration of 0.1 to 1,000,000 µM, preferably from 1 to 500,000 µM, and even more preferably from 10 to 100,000 µM. When more than one biocide is used at the same time, then the preferred concentration ranges above refer to each biocide that is being used. In the method of the present invention, to wt.% ratio of biocide to compound according to Formula (1) is preferably 5,000,000 or less, more preferably 1,500,000 or less and most preferably 500,000 or less. When more than one biocide and/or more than one compound according to Formula (1) are used, then the preferred wt.% ranges are based on the total amount of biocide and the total amount of compounds according to Formula (1) that are being used.

In one aspect the present invention relates to a biocide composition comprising one or more biocides and one or more compounds according to Formula (1). Preferably, the biocide formulation contains at least 2 wt.% biocide based on the total weight of the composition. The biocide composition may comprise 5-50 wt.% biocide, 10-25 wt.% biocide or 15-25 wt.% biocide. If the composition comprises more than one biocide, then the wt.% ranges are based on the total amount of biocide in the composition. Preferably, the biocide formulation contains at least 2 wt.% biocide based on the total weight of the composition.

The biocides suitable for use in the present invention may be generally divided into two groups: oxidizing and non-oxidizing (or conventional) biocides. The non-oxidizing biocides include biocides such as DNBPA, glutaraldehyde, isothiazolones, etc. One example of suitable biocides for use in the formulations of the invention includes non-oxidizing biocides. The biocides may also be divided into groups by function mechanisms. The electrophiles include oxidants, such as halogens and peroxy compounds, and electrophiles, such as formaldehyde, formaldehyde-releasers, isothiazolones, Bronopol and Cu, Hg and Ag. The membrane active biocides include lytic biocides, such as quats, biguanides, phenols and alcohols, and protonophores, such as parabens, weak acids and pyrithiones. Examples of non-oxidizing biocides include glutaraldehyde, 2,2-dibromo-3-nitrilopropionamide (DBNPA), 2-bromo-2-nitropropane-1,3-diol (Bronopol), 5-chloro-2-methyl-4-isothiazolin-3-one (CMIT), 2-methyl-4-isothiazolin-3-one (MIT), a mixture of CMIT and MIT, 1,2-dibromo-2,4-dicyanobutane, bis(trichloro-methyl)sulfone, 2-bromo-2-nitrostyrene, 4,5-dichloro-1,2-dithiol-3-one, 2-n-octyl-4-isothiazolin-3-one, 1,2-benzisothiazolin-3-one, ortho-phthalaldehyde, guanidines, biguanidines, pyrithiones, carbamates, 3-iodopropynyl-N-butylcarbamate, phosphonium salts such as tetrakis hydroxymethyl phosphonium sulfate (THPS), 3,5-dimethyl-1,3,5-thiadiazinane-2-thione (Dazomet), 2-(thiocyanomethylthio) benzo-thiazole, methylene bisthiocyanate (MBT), and combinations thereof.

Preferred biocides for the purpose of this invention are DBNPA (2,2-dibromo-3-nitrilopropionamide), glutaraldehyde, ADBAC (7.5% alkyl dimethyl benzyl ammonium chloride), THPS (tetrakis(hydroxymethyl)phosphonium sulfate), Bronopol (2-bromo-2-nitropropane-1,3-diol), DMO (dimethyloxazolidine), CTAC (1-(3-chloroallyl)-3,5,7-triaza-1-azoniaadamantane chloride), THNM (tris(hydroxymethyl)nitromethane), Dazomet, TTPC (Tributyl Tetradecyl Phosphonium Chloride), or other allowed biocide compounds in the different industries of interest. Additionally, further active agents can be included in the composition if required to achieve several functions such as anti-corrosion, biocidal effect, and antifoaming heat and pressure stability.

The present invention is now illustrated in greater detail by way of the following Examples, but it should be understood that the present invention is not deemed to be limited thereto.

### EXAMPLES

### Synthesis of a compound according to Formula (1)

Isosorbide digallate ester was synthesized in two steps from isosorbide and 3,4,5-tribenzyloxybenzoyl chloride as follows (3,4,5-tribenzyloxybenzoyl chloride was prepared as previously reported in Malik et al., Chemistry A European Journal 2012, 18(29), 9063-9074): 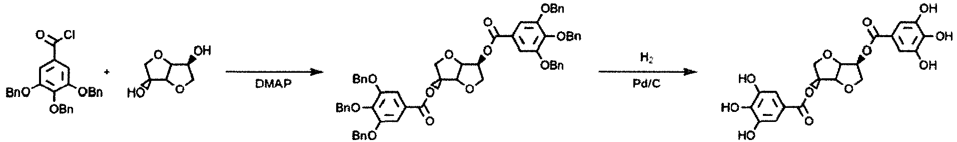 *Step 1*. In a nitrogen purged three necks flask fitted with a gas adaptor, a condenser and an addition funnel; 2.19 g isosorbide (15 mmol) and 4.59 g 4-dimethylaminopyridine (37.5 mmol - 2.5 eq.) were dissolved in 50 ml anhydrous toluene. A solution of 14.47 g 3,4,5-tribenzyloxybenzoyl chloride (31.5 mmol - 2.1 eq.) in 50 ml anhydrous toluene was added dropwise to this solution under nitrogen atmosphere over 30 min. Once the addition completed, the cloudy mixture was heated and maintained at reflux for 5h. After cooling, the dark mixture was filtered, washed twice with 100 ml brine, dried over MgSO₄ and concentrated under reduced pressure leaving 12.4 g (83%) of the benzyl protected diester compound as a pale yellow solid which can be further purified by chromatography on silica gel. This product was used for next step without further purification.

*Step 2.* In a nitrogen purged Schlenk flask, 1.5 g of the benzyl protected diester compound (1.5 mmol) was dissolved in 45 ml of MeOH/DCM mixture (1 to 2 volume ratio). 0.15 g of Pd/C (10 wt.%) was added and the mixture was stirred under H₂ atmosphere for 8h at room temperature. Next, the reaction mixture was filtered and the solvent was evaporated under reduced pressure leaving analytically pure isosorbide digallate ester.

### Biofilm eradicating effect

For biofilm inhibition tests, the following saline-based samples were prepared:

| Control | Saline solution |
|---|---|
| Sample A | 20 mM isosorbide digallate ester + 20 mM tetracycline |
| Sample B | 20 mM tetracycline |
| Sample C | 20 mM isosorbide + 40 mM gallic acid + 20 mM tetracycline |

All samples were tested in an *in vitro* model as described in Brackman et al., Journal of applied microbiology 2013, 114(6), 1833-1842 in order to determine the biofilm inhibitory effect of each samples. Briefly, *P. aeruginosa* grown overnight in solution were seeded in an artificial matrix and allowed to grow for 24h at 37°C to form a biofilm. The medium was then added with compounds at mentioned concentration and put back at 37°C for another 24 hr. After this the medium was removed, biofilms were washed with PS and then put into saline.

After sonication and vortexing, suspensions were plated and the resulting CFUs/biofilms was determined. The results are shown in Table 4 and Figure 1.

**Table 4**

| **Composition** | **CFU/AD** | **Log CFU/AD** |
|---|---|---|
| **Control (no treatment)** | 5.09 x 10⁹ | 9.71 |
| **Sample A** | < DL | < DL |
| **Sample B** | 2.34 x 10³ | 3.37 |
| **Sample C** | 1.02 x 10² | 2.01 |

| | | |
|---|---|---|
| *DL = detection limit | | |

Compositions A, B and C provided a marked biofilm eradicating effect. Surprisingly, Composition A comprising isosorbide digallate ester had the greatest biofilm eradicating effect and a superior activity than that of composition C composed of the individual components isosorbide and gallic acid.

### INDUSTRIAL APPLICABILITY

The present invention provides compounds, formulations and methods for preventing or treating bacteria biofilms, particularly in the oil and gas industries, paper industry, energy industry including power plants, water treatment industry. The present invention is particularly useful for preventing or treating bacteria biofilms in pipes, membranes, filters, pumps and other equipment used in these industries.

## Claims

**1.** A method for inhibiting or reducing the growth of a microbial biofilm on a surface, **characterised in that** the method comprises a step of contacting the biofilm-forming microorganism with a compound according to Formula (1), or a salt or solvate thereof: 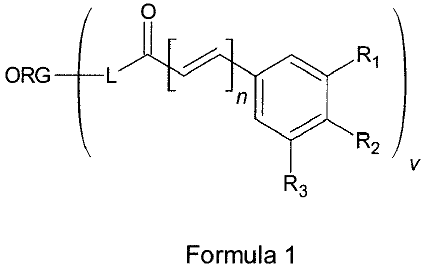 wherein
"ORG" is an organic moiety having from 2 to 20 carbon atoms;
"v" is 1,2, 3, 4, or 5;
"L" provides a covalent link to "ORG" and is selected from O, NH, or is a direct bond from the carbonyl group to "ORG";
"n" is 0 or 1; and
R₁, R₂ and R₃ are independently selected from H, OH and OCH₃ provided that at least one of R₁, R₂ and R₃ is OH.

**2.** The method according to claim 1, for inhibiting or reducing the growth of a microbial biofilm in an industrial water system, wherein the compound according to Formula (1), or a salt or solvate thereof, is introduced into the industrial water system.

**3.** The method according to claim 2, wherein the industrial water system is selected from a papermaking water system; a cooling water system such as a cooling tower, an open and/or closed loop cooling unit; an industrial raw water system; a drinking water distribution system; a sanitizing drinking water system; an oil production or recovery system such as an oil field water system or a system that comes into contact with drilling fluids; a fuel storage and/or transport system; a metal working system; a heat exchanger; a reactor; equipment used for storing and handling liquids; a boiler; a steam generating unit; a radiator; a flash evaporating unit; a refrigeration unit; reverse osmosis equipment; an ion selective membrane; a gas scrubbing unit; a blast furnace; a sugar evaporating unit; a steam power plant; a geothermal unit; a nuclear cooling unit; a water treatment unit; a pool recirculating unit; a mining circuit; a closed loop heating unit; a system having a surface that comes into contact with machining fluids used in operations such as drilling, boring, milling, reaming, drawing, broaching, turning, cutting, sewing, grinding, thread cutting, shaping, spinning and rolling; a system having a surface that comes into contact with hydraulic fluids; and ; a system having a surface that comes into contact with cooling fluids.

**3.** The method according to any one of claims 1 to 3, wherein the compound according to Formula (1) or a salt or solvate thereof, is used concomitantly with a biocide.

**4.** The method according to claim 4, wherein the biocide is selected from one or more of the following: chlorine, bromine, ozone, hydrogen peroxide, magnesium peroxide, glutaraldehyde, quaternary ammonium compounds, isothiazolones such as isothiazolin-3-one, methylene bisthiocyanate (MBT), phosphonium salts such as tetrakis hydroxymethyl phosphonium sulfate (THPS), tributyl tetradecyl phosphonium chloride (TTPC), Bronopol, 2,2-dibromo-3-nitrilopropionamide (DBNPA), 2-bromo-2-nitropropane-1,3-diol, 5-chloro-2-methyl-4-isothiazolin-3-one (CMIT), 2-methyl-4-isothiazolin-3-one (MIT), 1,2- dibromo-2,4-dicyanobutane, bis(trichloromethyl)sulfone, 2-bromo-2-nitrostyrene, 4,5-dichloro-1,2-dithiol-3-one, 2-n-octyl-4-isothiazolin-3-one, 1,2-benzisothiazolin-3-one, ortho-phthalaldehyde, guanidines, biguanidines, pyrithiones, carbamates, 3-iodopropynyl-N-butylcarbamate, 3,5-dimethyl-1,3,5-thiadiazinane-2-thione, and 2-(thiocyanomethylthio)benzothiazole.

**5.** The method according to any one of claims 1 to 4, wherein "v" in Formula (1) is 2, 3, or 4.

**6.** The method according to any one of claims 1 to 5, wherein:
"n" in Formula (1) is 0 for at least one moiety as defined within the round bracket; or
"n" in Formula (1) is 1 for at least one moiety as defined within the round bracket; or
"L" in Formula (1) is O for at least one moiety as defined within the round bracket; or
"L" in Formula (1) is NH for at least one moiety as defined within the round bracket.

**7.** The method according to any one of claims 1 to 6, wherein for at least one moiety as defined within the round bracket in Formula (1) R₂ is OH and at least one of R₁ and R₃ is OH or OCH₃.

**8.** The method according to any one of claims 1 to 7, wherein for at least one moiety as defined within the round bracket in Formula (1) "n", R₁, R₂ and R₃ are as follows:
(i) "n" is 1 and R₁ is H, R₂ is OH, and R₃ is H ; or
(ii) "n" is 1 and R₁ is OH, R₂ is OH, and R₃ is H; or
(iii) "n" is 1 and R₁ is OCH₃, R₂ is OH, and R₃ is H; or
(iv) "n" is 1 and R₁ is OCH₃, R₂ is OH, and R₃ is OH; or
(v) "n" is 1 and R₁ is OCH₃, R₂ is OH, and R₃ is OCH₃; or
(vi) "n" is 0 and R₁ is OH, R₂ is OH, and R₃ is H; or
(vii) "n" is 0 and R₁ is OCH₃, R₂ is OH, and R₃ is H; or
(viii) "n" is 0 and R₁ is OH, R₂ is OH, and R₃ is OH; or

**9.** The method according to any one of claims 1 to 8, wherein "v" in Formula (1) is 2, 3, or 4 and wherein "ORG" in Formula (1) is a linear or branched C2-C5 alkane, linear or branched C2-C5 alkene, or linear or branched C2-C5 alkyne that, in addition to the moiety satisfying the structure within the round brackets of Formula (1), may optionally be substituted with one or more substituents selected from -OH; -NH₂; -NH₃⁺X where X is F, Cl, Br or I; a halogen selected from F, Cl, Br or I; -NO₂; a C6-C10 aryl which is linked to the alkane, alkene or alkyne via a linking group selected from direct bond, -O-, -CO-, -COO-,-OCOO-, -COOCH₂-, -COOC₂H₄-, -COOCH=CH-, -NH-, -CONH-, -COONH-, -CSO-, or-CSS-; a 5 to 10 membered saturated, unsaturated or aromatic heterocycle containing 1 to 4 heteroatoms selected from O, N and S which is linked to the alkane, alkene or alkyne via a linking group selected from direct bond, -O-, -CO-, -COO-, -OCOO-, -COOCH₂-,-COOC₂H₄-, -COOCH=CH-, -NH-, -CONH-, or -COONH-; and wherein the aforementioned aryl and heterocycle groups may optionally carry one, two or three substituents independently selected from -OH, F, Cl, Br, I, -NO₂, -NH₂, -NH₃⁺X where X is F, Cl, Br or I.

**10.** The method according to any one of claims 1 to 8, wherein "ORG" in Formula (1) is a saturated C5-C6 monocyclic or bicyclic heterocycle having one or two oxygen heteroatoms and that, in addition to the moiety satisfying the structure within the round brackets of Formula (1), may optionally be substituted with one or more substituents selected from -OH;-NH₂; -NH₃⁺X where X is F, Cl, Br or I; a halogen selected from F, Cl, Br or I; -NO₂; a C6-C10 aryl which is linked to the alkane, alkene or alkyne via a linking group selected from direct bond, -O-, -CO-, -COO-, -OCOO-, -COOCH₂-, -COOC₂H₄-, -COOCH=CH-, -NH-, - CONH-, -COONH-, -CSO-, or -CSS-; a 5 to 10 membered saturated, unsaturated or aromatic heterocycle containing 1 to 4 heteroatoms selected from O, N and S which is linked to the alkane, alkene or alkyne via a linking group selected from direct bond, -O-, -CO-, -COO-,-OCOO-, -COOCH₂-, -COOC₂H₄-, -COOCH=CH-, -NH-, -CONH-, -COONH-, -CSO-, or-CSS-; and wherein the aforementioned aryl and heterocycle groups may optionally carry one, two or three substituents independently selected from -OH, F, Cl, Br, I, -NO₂, -NH₂, -NH₃⁺X where X is F, Cl, Br or I

**11.** The method according to any one of claims 1 to 10, wherein "ORG" in Formula (1) is represented by one of the following structures: 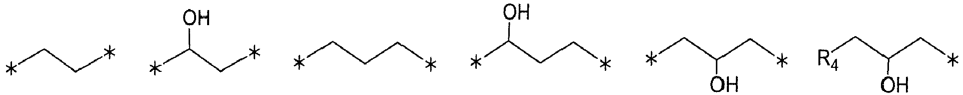 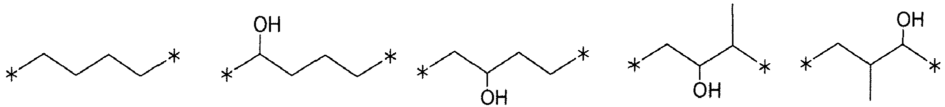 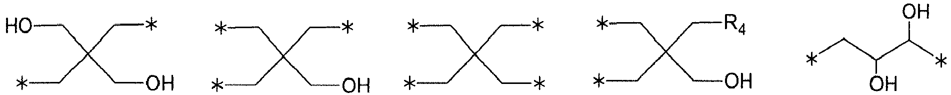 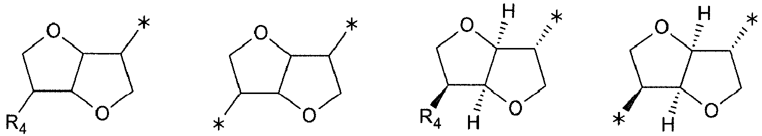
wherein, the asterisk denotes the position of "L" within the round bracket in Formula (1); and
R₄ is a substituent selected from -OH; -NH₂; -NH₃⁺X where X is F, Cl, Br or I; a halogen selected from F, Cl, Br or I; -NO₂; a C6-C10 aryl which is linked to the alkane, alkene or alkyne via a linking group selected from direct bond, -O-, -CO-, -COO-, -OCOO-,-COOCH₂-, -COOC₂H₄-, -COOCH=CH-, -NH-, -CONH-, -COONH-, -CSO-, or -CSS-; a 5 to 10 membered saturated, unsaturated or aromatic heterocycle containing 1 to 4 heteroatoms selected from O, N and S which is linked to the alkane, alkene or alkyne via a linking group selected from direct bond, -O-, -CO-, -COO-, -OCOO-, -COOCH₂-, -COOC₂H₄-,-COOCH=CH-, -NH-, -CONH-, -COONH-, -CSO-, or -CSS-; and wherein the aforementioned aryl and heterocycle groups may optionally carry one, two or three substituents independently selected from -OH, F, Cl, Br, I, -NO₂, -NH₂, -NH₃⁺X where X is F, Cl, Br or I.

**13.** The method according to any one of claims 1 to 11, wherein the compound according to Formula (1) has one of the following structures:

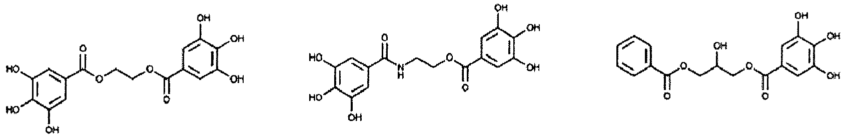

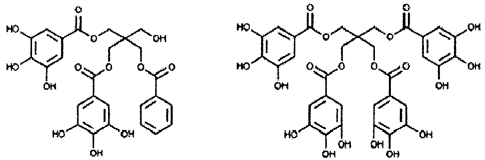

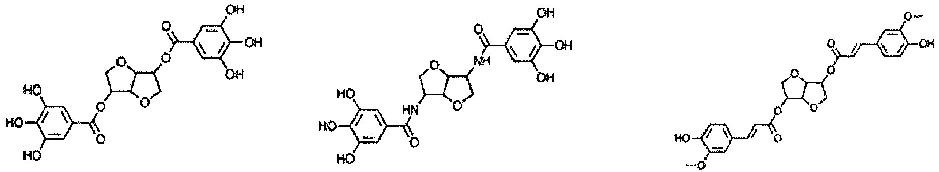

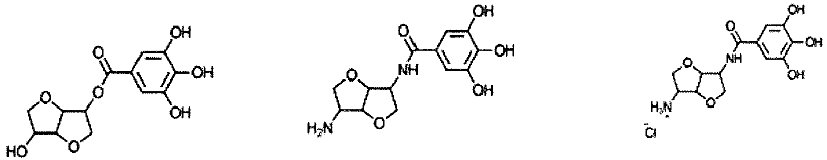

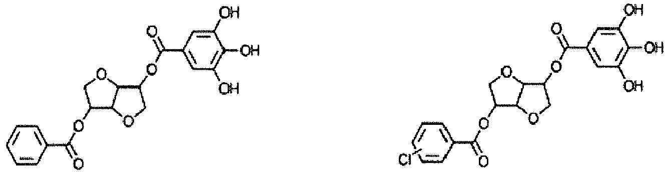

**14.** A compound according to Formula (1), or a salt or solvate thereof: 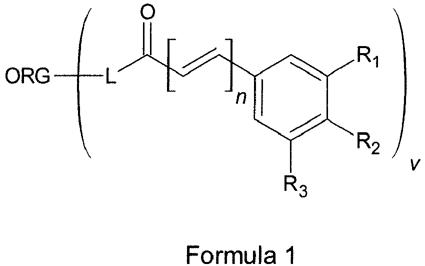 wherein
"v"is 1, 2, 3, 4, or 5;
"L" provides a covalent link to "ORG" and is selected from O, NH, or is a direct bond from the carbonyl group to "ORG";
"n" is 0 or 1;
R₁, R₂ and R₃ are independently selected from H, OH and OCH₃ provided that at least one of R₁, R₂ and R₃ is OH; and
"ORG" is an organic moiety having from 2 to 20 carbon atoms and selected from one of the following structures: 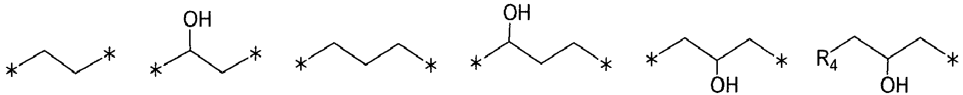 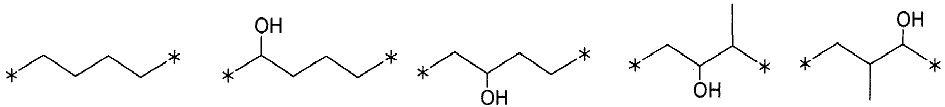 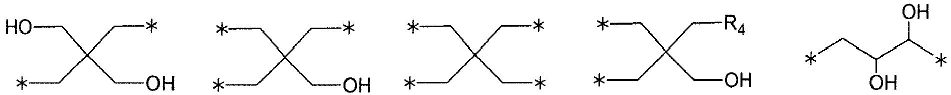 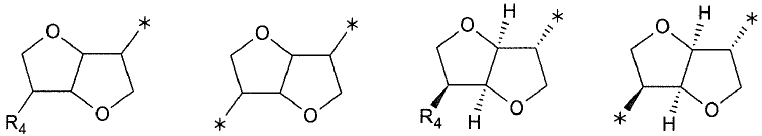
wherein, the asterisk denotes the position of "L" within the round bracket in Formula (1); and
R₄ is a substituent selected from -OH; -NH₂; -NH₃⁺X where X is F, Cl, Br or I; a halogen selected from F, Cl, Br or I; -NO₂; a C6-C10 aryl which is linked to the alkane, alkene or alkyne via a linking group selected from direct bond, -O-, -CO-, -COO-, -OCOO-,-COOCH₂-, -COOC₂H₄-, -COOCH=CH-, -NH-, -CONH-, -COONH-, -CSO-, or -CSS-; a 5 to 10 membered saturated, unsaturated or aromatic heterocycle containing 1 to 4 heteroatoms selected from O, N and S which is linked to the alkane, alkene or alkyne via a linking group selected from direct bond, -O-, -CO-, -COO-, -OCOO-, -COOCH₂-, -COOC₂H₄-,-COOCH=CH-, -NH-, -CONH-, -COONH-, -CSO-, or -CSS-; and wherein the aforementioned aryl and heterocycle groups may optionally carry one, two or three substituents independently selected from -OH, F, Cl, Br, I, -NO₂, -NH₂, -NH₃⁺X where X is F, Cl, Br or I.

**15.** A biocide composition comprising a biocide and a compound to Formula (1) or salt or solvate thereof,
wherein the biocide is selected from one or more of the following: chlorine, bromine, ozone, hydrogen peroxide, magnesium peroxide, glutaraldehyde, quaternary ammonium compounds, isothiazolones such as isothiazolin-3-one, methylene bisthiocyanate (MBT), phosphonium salts such as tetrakis hydroxymethyl phosphonium sulfate (THPS), tributyl tetradecyl phosphonium chloride (TTPC), Bronopol, 2,2-dibromo-3-nitrilopropionamide (DBNPA), 2-bromo-2-nitropropane-1,3-diol, 5-chloro-2-methyl-4-isothiazolin-3-one (CMIT), 2-methyl-4-isothiazolin-3-one (MIT), 1,2- dibromo-2,4-dicyanobutane, bis(trichloromethyl)sulfone, 2-bromo-2-nitrostyrene, 4,5-dichloro-1,2-dithiol-3-one, 2-n-octyl-4-isothiazolin-3-one, 1,2-benzisothiazolin-3-one, ortho-phthalaldehyde, guanidines, biguanidines, pyrithiones, carbamates, 3-iodopropynyl-N-butylcarbamate, 3,5-dimethyl-1,3,5-thiadiazinane-2-thione, and 2-(thiocyanomethylthio)benzothiazole; and
wherein the compound according to Formula (1) has the following structure: 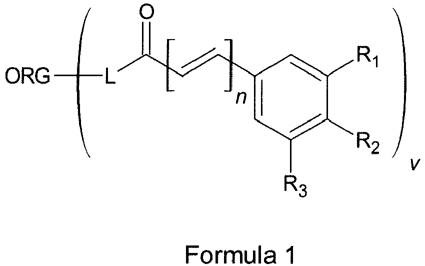 wherein
"ORG" is an organic moiety having from 2 to 20 carbon atoms;
"v" is 1, 2, 3, 4, or 5;
"L" provides a covalent link to "ORG" and is selected from O, NH, or is a direct bond from the carbonyl group to "ORG";
"n" is 0 or 1; and
R₁, R₂ and R₃ are independently selected from H, OH and OCH₃ provided that at least one of R₁, R₂ and R₃ is OH.
